Europäisches Patentamt

European Patent Office

Office européen des brevets

(11) Publication number: **0 359 489**
**A2**

# EUROPEAN PATENT APPLICATION

(21) Application number: **89309158.7**

(22) Date of filing: **08.09.89**

(51) Int. Cl.5: **A61M 25/10**

(30) Priority: **15.09.88 US 244978**

(43) Date of publication of application:
**21.03.90 Bulletin 90/12**

(84) Designated Contracting States:
**DE FR GB**

(71) Applicant: **BAXTER INTERNATIONAL INC.**
**One Baxter Parkway**
**Deerfield, IL 60015(US)**

(72) Inventor: **Bacich, Steven Robert**
**28351 La Falda Street**
**Laguna Niguel, CA 92677(US)**
Inventor: **Chin, Albert K.**
**3885 Nelson Drive**
**Palo Alto, CA 94306(US)**
Inventor: **Lowery, Guy Russell**
**26781 Magdalena Lane**
**Mission Viejo, CA 92691(US)**

(74) Representative: **MacGregor, Gordon et al**
**ERIC POTTER & CLARKSON 14 Oxford Street**
**Nottingham, NG1 5BP(GB)**

(54) **Everting balloon catheter with anchor annulus and balloon for same.**

(57) Disclosed is an everting thru-lumen balloon catheter (1) having an outer tubular unit (2) defining a central axis and an outer lumen (10), which unit also has a distal (28) and proximal tip and an interior and exterior surface. Disposed within the outer unit is a movable inner tube (6) coaxial with the outer unit and having a distal and proximal tip. An expandable tubular balloon (36) having a first and second end is attached at its first end to the distal tip of the inner tube, attached at its second end to the exterior surface of the outer unit spaced about a centimeter from the distal tip (28), and made from a material sufficiciently pliable to inflate at about 2 atmospheres pressure and sufficiently strong to withstand about 10 to about 12 atmospheres of pressure so that upon inflation of the balloon through the outer lumen, expansion of the balloon occurs radially with respect to the axis of the outer unit. The balloon is preferably made of a cross-linked copolymer of vinyl acetate and polyethylene having about 18 percent vinyl acetate monomer units. Also disclosed s a catheter balloon of the above composition and a composition for such balloons.

*FIG. 1*

## EVERTING BALLOON CATHETER WITH ANCHOR ANNULUS AND BALLOON FOR SAME

### BACKGROUND OF THE INVENTION

Field of the Invention

This invention relates to catheters and materials for catheters, particularly everting thru-lumen balloon catheters with flexible balloons. The invention is particularly useful in catheters for dilatation, the inflation and/or repeated inflation of a balloon within a plaque occlusion or stenosis to reform the plaque deposit against the vessel wall and open the vessel.

Description of the Prior Art

Balloon catheters and everting balloon catheters are known in the art. For example, as disclosed in U.S. Patent Nos. 4,437,857, 4.604,094 and 4,530,698, method and apparatus for everting a balloon using a balloon material substantially more flexible than that of the catheter body is known. Flexible but non-elastomeric balloon materials such as polyethylene are used for the balloons as disclosed in U.S. Patents Nos. 4,493,711 and 4,606,347, and polyurethane blends have been suggested for some balloons, but will not withstand the pressures required for dilatation.

Methods and apparatus for everting a tube are shown in U.S. Patent No. 3,506,011. As shown in U.S. Patent 4,271,839, the use of a balloon attached to slidable thru-lumen tubing or reinversion member at one end of the balloon and to the catheter body at the other end of the balloon so that the balloon is everted by sliding the thru-lumen tubing distally is known for dilatation. A problem, though, with standard designs for such a thru-lumen everting balloon is that upon eversion, the catheter is pushed backwards away from the occlusion, so that it is difficult to locate the catheter at the appropriate spot adjacent the lesion for dilatation. Furthermore, higher pressures are required for eversion of the balloon than may be desired for dilatation, so that the balloon must be deflated before dilatation.

Balloons are frequently attached to the tips of catheters to anchor them in place in the vascular system; one way of solving the above-mentioned difficulty in locating the balloon within the lesion is shown in U.S. Patent No. 4,630,609, which teaches the use of a separate anchor balloon to anchor an everting balloon catheter in place.

The use of an internal, slidable, tubular sheath to enhance the eversion process is shown in EPO Patent Application 86630101.1 and a seal for the thru-lumen is shown In U.S. Patent No. 4,606,347. Also, U.S. Patents No. 4,526,175 and 4,689,041 show different balloon structures.

### SUMMARY OF THE INVENTION

The present invention in one aspect includes an improved thru-lumen everting catheter with an anchor annulus and material for the balloon of same. The balloon of the catheter everts at relatively low pressures and is subject to minimized backward force during eversion, so that anchoring the catheter at the lesion is significantly simpler.

In another aspect, the invention includes a balloon apparatus for accessing and anchoring in a circumscribed area. The apparatus includes an outer tubular unit defining a central axis and an outer lumen and having a distal and proximal tip and an interior and exterior surface. It also includes a movable inner tube coaxial with the outer unit and having a distal and proximal tip, and an expandable tubular balloon having a first and second end. The balloon is attached at its first end to the distal tip of the inner tube, and at its second end to the exterior surface of the outer unit spaced from the distal tip so that upon inflation of the balloon through the outer lumen, expansion of the balloon occurs radially with respect to the axis of the outer unit.

Preferably, the balloon is spaced from the distal tip of the outer unit by about 1 centimeter and the balloon is attached to the outer unit in a recession so that the diameter of the outer unit is substantially the same at the point of attachment as elsewhere. Also, the balloon is preferably comprised of a material sufficiently pliable to inflate at pressures lower than about 3, preferably at about 2 atmospheres, yet sufficiently strong to withstand pressures of more than about 8 preferably at least about 10 to 12 atmospheres. The material is usually a cross-linked copolymer of vinyl acetate and polyolefins, usually polyethylene, wherein the material has about 1 to 51, preferably about 10 to 30 and most preferably about 18 percent of vinyl acetate monomer units. In another aspect the invention includes a catheter balloon comprised of the materials described above and the composition for such a balloon.

### BRIEF DESCRIPTION OF THE DRAWINGS

Fig. 1 is a cross-section of the tip of the preferred catheter of the present invention with the balloon fully inflated and everted.

Fig. 2 is a front elevation of the preferred catheter of the present invention with a cut-away section of the tip showing the balloon fully inverted.

Figs 3A and 3B are cross-sections of the tips of prior everting thru-lumen catheters showing inflation and eversion of the balloons.

Fig. 3C is a cross-section of the tip of the preferred catheter of the present invention, particularly showing inflation and eversion of the balloon of the preferred present catheter to form an anchor annulus anchoring the catheter in place for dilation.

Figs. 4A through 4D and 4A′ through 4D′, respectively, are cross-sections of prior catheters showing inflation and eversion of the balloons, and the corresponding saline pressures required for inflation and eversion.

Figs. 5A through 5D and 5A′ through 5D′, respectively, are cross-sections of the tip of the preferred present catheter, showing inflation and eversion and the pressure requirements for inflation and inversion.

Fig. 6 is a cross-section of the catheter taken at section 6-6 of Fig. 1, illustrating the lumens and interior structure of the catheter.

## DESCRIPTION OF THE PREFERRED EMBODI-
## MENTS

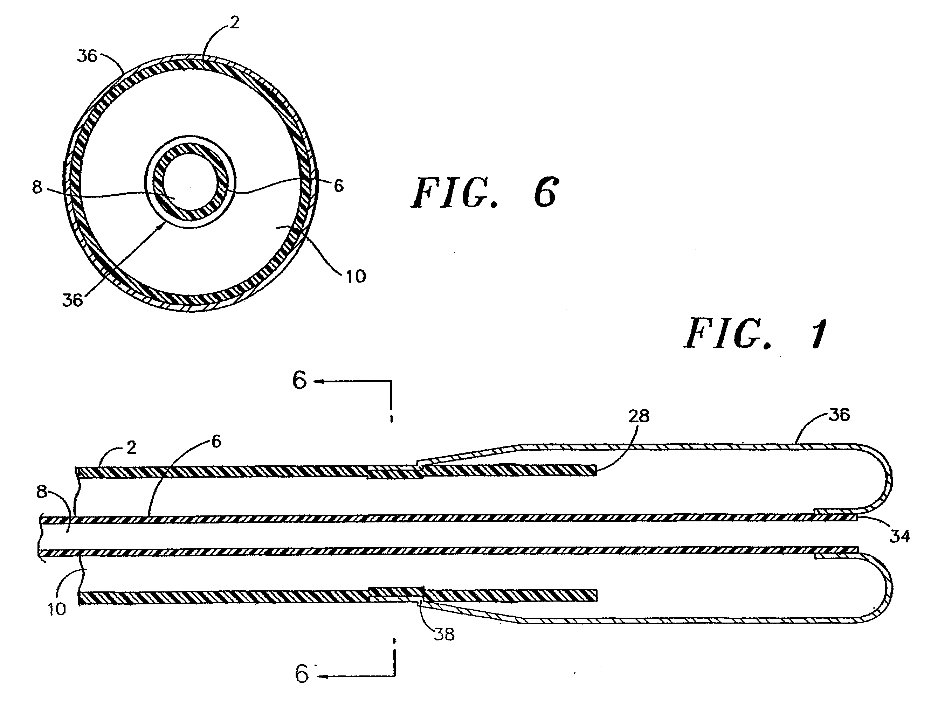

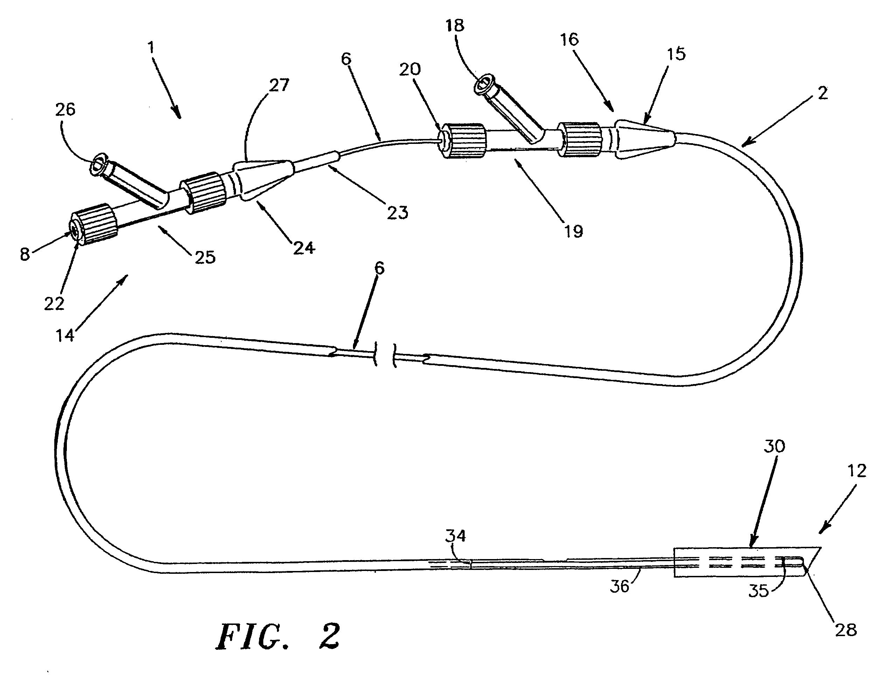

Referring now to Figs. 1 and 2, the preferred embodiment of the everting catheter 1 is a dilatation catheter including an outer tube unit 2 about 0.06 inches in outer diameter and about 135 cm. in length defining a central axis. The catheter has a central or inner tube 6 coaxial with the outer tube unit to form a double lumen catheter preferably about 150 cm. long, one lumen 8 (the inner or "thru-lumen") axially disposed within the other 10, as shown in Fig. 6. The thru-lumen is as long as the outer tube 2 plus the length of balloon 36, although additional length can be added for easy maneuverability during eversion and reinversion. The thru-lumen 8 is open to the environment at the distal end 12 of the catheter and tubing 6 is preferably about 0.02 inches in inner diameter and about 0.028 inches in outer diameter. The thru-lumen allows use of the catheter over a guidewire, and, via injection port 26, can be used to inject dye, contrast media, saline, or other fluids into the system.

The catheter 1 terminates at proximal end 14 in an outer hub 16 with wings 15. The outer hub is attached to an outer Y connector 19 having an inflation port 18 accessing outer lumen 10. Through an "O" ring 20 sealing outer lumen 10 from the environment at the proximal end, inner or central tube 6 slideably extends proximally from outer hub 16 to terminate in a sleeve 23 surrounded by an inner hub 24 having wings 27 and attached to an inner Y-connector 25 having an injection or infusion port 26 which accesses the thru-lumen. Access to the inner lumen at the proximal end is gained through an O ring 22 which can form a seal.

Sheath 23 is usually formed of heatshrunk polyvinyl chloride ("PVC") and the two hubs and adapters 16 and 24 are usually formed of polycarbonate or PVC.

Preferably the outer tubing 2 of the catheter is formed of nylon and the inner tubing 6 of nylon or polyether sulfone. The outer tube may include a radiopaque dye such as Barium, which may also increase stiffness. The primary considerations for the material for the tubing are, of course, biological inertness, inertness to saline, contrast media and other materials to be infused through the tubing, and sufficient flexibility, resistance to deformity, and torquability to bend the catheter through the tortuous pathways of the vascular or any other system in which it will be used yet retain torquability without crimping or occlusion.

The catheter 1 terminates in a tip 28 covered while the catheter is stored in sterile condition before use by a cover or protective sleeve 30. A radiopaque marker 35 of stainless steel, tungsten/rhenium, or any other biocompatible metal or metal/polymer composite is formed annularly about outer tube 2 about a centimeter back from the distal tip 28 so that the position of the catheter in the vascular system can be easily identified when it is in use.

When central tube 6 is in its fully withdrawn position, its tip 34 usually terminates up to about 15 cm. back from the distal tip 28 of the device. The exact distance from tip 28 will vary with the particular purpose for which the catheter is designed and the length of the expected occlusion in the vessel; thus models for use in the coronary arteries may terminate about 2 to 4 cm back, while peripheral models may easily terminate 10 cm. or more back.

One end of tubular balloon 36 is attached to the periphery of central tube 6 at the distal end thereof; the other end of the balloon is inverted and attached to the exterior of outer tube 2 spaced usually about a centimeter back from the distal tip 28, in an annular recession 38 in the exterior of the outer tube. The balloon is bonded to recession 38 thermally or by adhesives; in some instances it may be textured on the surface to promote anchoring at the desired spot in the vessel. In some embodiments, the balloon and the tubing can be a unitary piece, so attachment of the balloon to the tubing need not be a concern.

When inner tube 34 is completely withdrawn, the balloon remains inside the catheter; when it is moved toward distal end 12 of the catheter, it causes the balloon to evert and aids balloon inflation when saline is simultaneously infused through the inflation port 18 to inflate the balloon 36.

The balloon is formed of a material more pliable than that of prior balloons made of polyethylene. It tends to inflate and evert at lower pressures than prior balloons and adapt better to the contours of the particular vessel. The material used In the dilatation catheter, however, has sufficient strength to sustain the pressures required for dilatation, usually about 10 to 12 atmospheres.

In the preferred embodiment, the balloon is formed of vinyl acetate blended with polyolefins in a copolymer product. Preferably, the polyolefins are polyethylene, and the copolymer contains about 1 to about 51 percent vinyl acetate monomer units, more preferably about 10 to about 30 percent vinyl acetate monomer units, and most preferably about 18 percent vinyl acetate monomer units. The preferred material is sold by DuPont under the trademark Elvax 460.

To make the catheter, the balloon material is extruded to form tubing and irradiated with electron-beam radiation in the fashion used for cross-linking standard balloons. Irradiation is usually done with a 15 milliamp electron beam at a 200 feet per minute line speed to produce a cross-linked copolymer. The balloons are then blown and cut to size.

The inner tubing material is also extruded and cut to size, and the balloon bonded to the outer distal tip thermally or preferably by using biocompatible adhesives such as cyanoacrylate or the urethane adhesives. A mandril is passed into the balloon which is inverted by hand rolling its open end back over the mandril and around the inner tube.

The outer tubing material is also obtained and compounded, then extruded and cut to size and the radiopaque marker attached. The inner tubing is backloaded into the outer tubing and the balloon bonded to the outer tubing by heatshrinking or the application of adhesives. In the preferred embodiment, heat is applied to the outer tubing to create recession 38 and a biocompatible adhesive used to attach the balloon.

The sheath is then heatshrunk about the proximal end of the inner tubing and the outer hub attached to the outer tubing using a cyanoacrylate adhesive, followed by the outer Y connector, the inner hub and the inner Y connector. The package is inspected and finally sterilized and sold.

In operation, the catheter 1 is introduced into the vascular system, usually via a guiding catheter, particularly when used for percutaneous coronary access. Where it will be inserted only into peripheral vessels or is inserted intraoperatively, usually no guiding catheter is used, although a guidewire can be used through the thru-lumen.

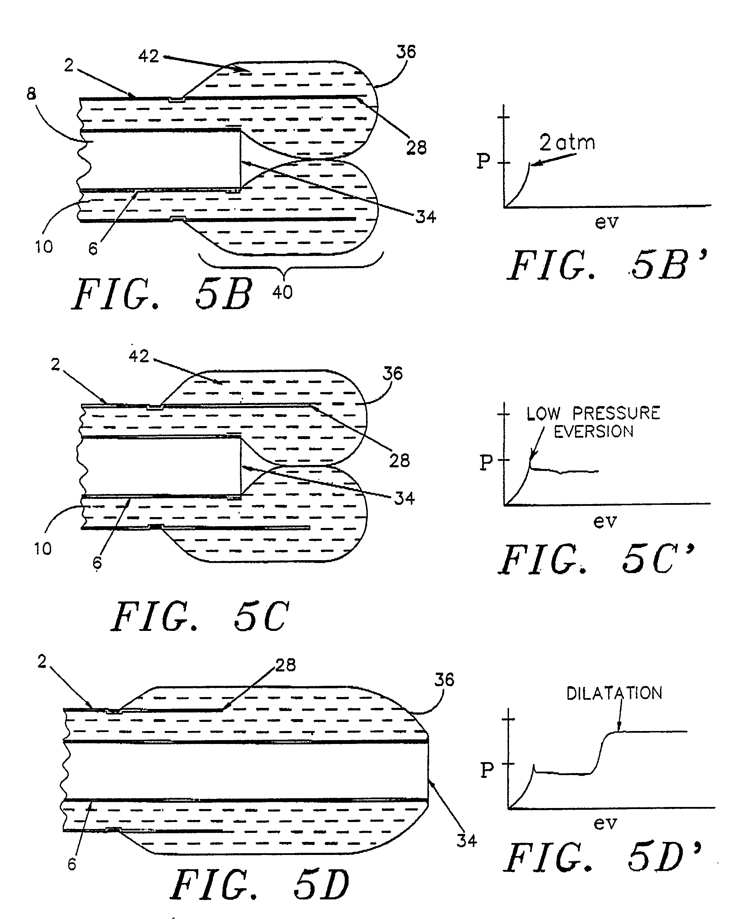

The tip of the catheter is inserted as close as possible to the lesion 52 to be removed, under fluoroscopy, and the balloon is inflated with saline through the inflation port 18 to about 2 atmospheres. Inner tube 6 is slid distally to evert the balloon 36 until resistance is felt, indicating that the balloon has formed an anchor annulus 42 (an inflated ring along the outside distal length 40 of the catheter which anchors the catheter in the lesion, see Figs. 5B and 5C). If the radiopaque marker continues to move, however, the catheter is insufficiently anchored, and the balloon inflated further until the anchor annulus is formed. The more compliant the balloon material, the lower the amount of pressure required to form the anchor annulus. Silicone texturing on the exterior of the annulus may further help to anchor the catheter.

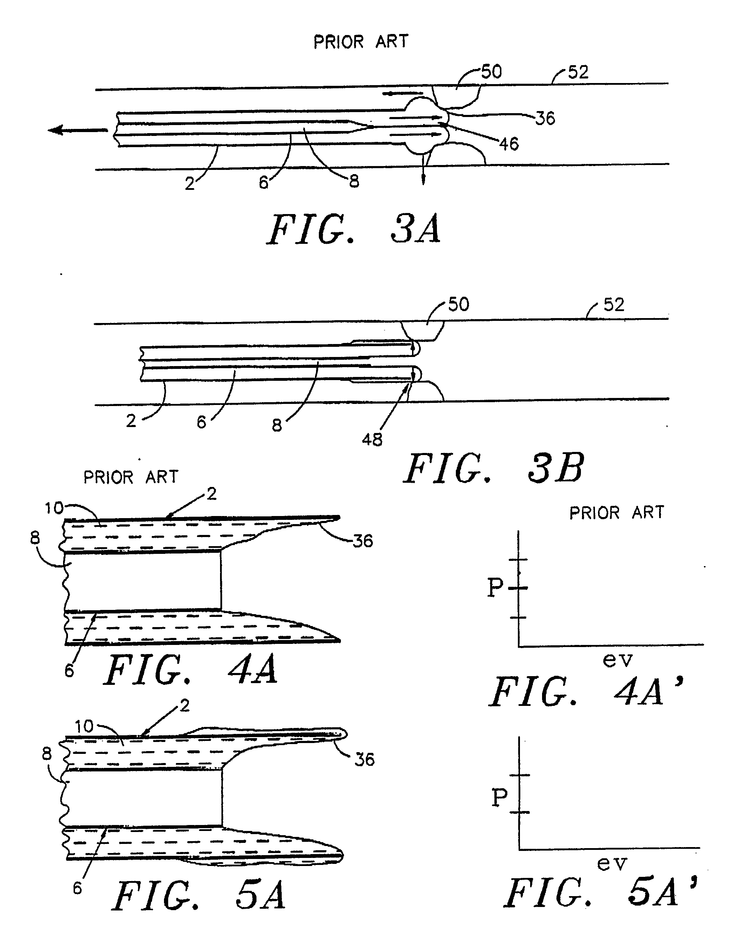

Pushing the inner tube distally, the balloon is then further everted to extend it until it crosses the stenosis, as shown in Figs 3B and 5D. At that point, dilatation (or any diagnostic purpose for which the catheter is inserted) is handled in the standard manner. When the process is finished, the balloon is deflated (via the inflation port) and the catheter withdrawn, or the balloon is reinverted by pulling the inner tube 6 proximally, and the catheter then is withdrawn.

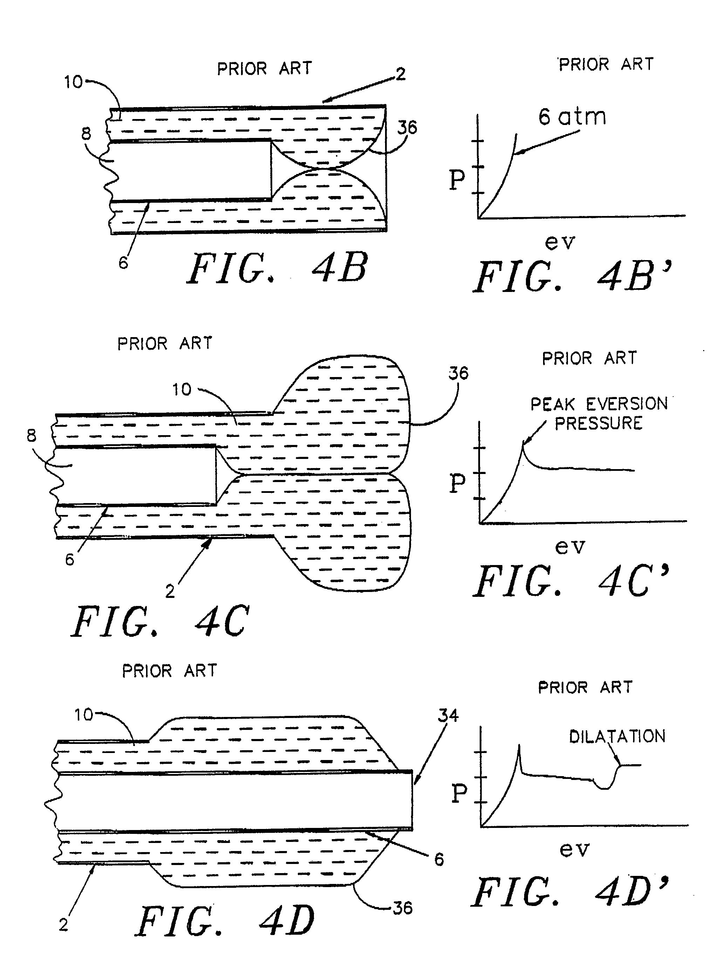

The present invention provides significant advantages over prior catheters. Inflation of an everting thru-lumen balloon catheter of the prior art, and the pressures required, are shown in Figs 4A through 4D, and 4A' through 4D'. When initially pressurized as shown in Figs. 4A and 4A', 4B and 4B', the typical prior catheter required about 6 atmospheres of pressure to initially inflate the balloon 36, at which point the balloon expanded into the thru-lumen only.

Upon further inflation, eversion began, the balloon everting at the end of the outer tube to form an essentially club-shaped balloon 36, as shown in Fig 4C. Inflation pressures within the balloon tended to produce a force 46 operating to extend the distal end of the balloon, and moving parallel to the inner and outer tubing. This tended to force the catheter backwards, away from the lesion, so that it was frequently necessary to deflate, reposition, and reinflate the balloon. Finally, the balloon was completely everted, but sometimes had to be partially deflated when the large initial pressure was too high for effective dilatation. Dilatation was then accomplished, and the balloon deflated and withdrawn.

The catheter of the present invention, however, as shown together with the pressure levels required in Figs 5A through 5D and 5A' through 5D', requires only about 2 atmospheres pressure to inflate initially and to obtain eversion, due to its particularly pliable balloon. When eversion begins, it results not in a balloon extending forward at the tip of the catheter, but in an extended, generally tubular balloon 36 which is inflated to extend along the outside length 40 of the catheter to form anchor annulus 42 as shown in Figs. 5B and 5C. Because the forces of inflation 48 within the balloon of this catheter tend to expand it radially with respect to the longitudinal axis of the catheter, the catheter is not forced backwards, but instead becomes anchored in place in the lesion.

Central tube 6 is slid distally to further evert the balloon 36 and an appropriate amount of inflation pressure added to fully (or partially, when appropriate) evert the balloon, as shown in Fig. 5D. Dilatation (or any other purpose for which the catheter was inserted) is then accomplished, the balloon being further inflated to appropriate pressures for dilatation if necessary.

Thus, the present invention requires much lower pressures than prior units to inflate and position the balloon; further, it is much easier to position the present catheter adjacent the lesion because the balloon tends to form an anchor annulus which anchors it in the lesion prior to eversion.

It should be understood that the foregoing description is intended by way of illustration and not by way of limitation and that many modifications and variations are within the scope of the invention which is described by the appended claims. Furthermore, it should be noted that the invention relates to many different types of apparatus and catheters, not just dilatation catheters.

## Claims

1. Balloon apparatus for accessing and anchoring in a circumscribed area comprising:
an outer tubular unit defining a central axis and an outer lumen, and having a distal and proximal tip and an interior and exterior surface;
a movable inner tube coaxial with the outer unit and having a distal and proximal tip;
an expandable tubular balloon having a first and second end, attached at its first end to the distal tip of the inner tube, and at its second end to the exterior surface of the outer unit spaced from the distal tip so that upon inflation of the balloon through the outer lumen, expansion of the balloon occurs radially with respect to the axis of the outer unit.

2. Apparatus according to claim 1 and wherein the balloon is spaced from the distal tip of the outer unit by about one-half centimeter to about 3 centimeters.

3. Apparatus according to claim 2 and wherein the balloon is spaced from the distal tip of the outer unit by about 1 centimeter.

4. Apparatus according to claim 1 and wherein the balloon is attached to the outer unit in a recession so that the diameter of the outer unit is substantially the same at the point of attachment as elsewhere.

5. Apparatus according to claim 1 and wherein the balloon is comprised of a material sufficiently pliable to inflate at pressures lower than about 3 atmospheres, yet sufficiently strong to withstand pressures of more than about 8 atmospheres.

6. Apparatus according to claim 5 and wherein the material is sufficiently pliable to inflate at pressures of about 2 atmospheres, yet sufficiently strong to withstand pressures of about 10 to about 12 atmospheres.

7. Apparatus according to claim 6 and wherein the material is a cross-linked copolymer of vinyl acetate and polyolefins having about 1 to 51 percent vinyl acetate monomer units.

8. Apparatus according to claim 7 and wherein the material has about 10 to 30 percent of vinyl acetate monomer units and the polyolefins consist essentially of polyethylene.

9. Apparatus according to claim 8 and wherein the material has about 18 percent of vinyl acetate monomer units.

10. A catheter balloon comprised of a sufficiently pliable material to inflate at pressures under about 3 atmospheres and sufficiently strong to withstand pressures of about 8 atmospheres or more.

11. A catheter balloon according to claim 10 and wherein the material inflates at pressures of about 2 atmospheres and withstands pressures of about 10 to about 12 atmospheres so that it can be used for dilatation catheters.

12. A catheter balloon according to claim 11 and wherein the material is a cross-linked copolymer of vinyl acetate and polyolefins having about 1 to about 51 percent vinyl acetate monomer units.

13. A catheter balloon according to claim 12 and wherein the copolymer has about 10 to about 30 percent vinyl acetate monomer units and the polyolefins consist essentially of polyethylene.

14. A catheter balloon according to claim 13 and wherein the copolymer has about 18 percent vinyl acetate monomer units.

15. A composition for a catheter balloon consisting essentially of a cross-linked copolymer of vinyl acetate and polyolefins having about 1 to about 51 percent vinyl acetate monomer units.

16. A composition for a catheter balloon according to claim 15 wherein the copolymer has about 10 to about 30 percent vinyl acetate monomer units and the polyolefins are polyethylene.

17. A composition for a catheter balloon according to claim 16 wherein the copolymer has about 18 percent vinyl acetate monomer units.

18. An everting thru-lumen balloon catheter comprising:
an outer tubular unit defining a central axis and an outer lumen, and having a distal and proximal tip and an interior and exterior surface;
a movable inner tube coaxial with the outer unit and having a distal and proximal tip;
an expandable tubular balloon having a first and second end, said balloon attached at its first end to the distal tip of the inner tube, attached at its second end to the exterior surface of the outer unit spaced about a centimeter from the distal tip, and made from a material sufficiciently pliable to inflate at about 2 atmospheres pressure and sufficiently strong to withstand about 10 to about 12 atmospheres of pressure so that upon inflation of the balloon through the outer lumen, expansion of the balloon occurs radially with respect to the axis of the outer unit.

19. A catheter according to claim 18 and wherein the material is a cross-linked copolymer of vinyl acetate and polyethylene having about 18 percent vinyl acetate monomer units.

FIG. 1

FIG. 6

FIG. 2

EP 0 359 489 A2

PRIOR ART

FIG. 3A

FIG. 3B

PRIOR ART

FIG. 4A

PRIOR ART

FIG. 4A'

FIG. 5A

FIG. 5A'

PRIOR ART

PRIOR ART

*FIG. 4B*

6 atm

P

ev

*FIG. 4B'*

PRIOR ART

PRIOR ART

*FIG. 4C*

PEAK EVERSION
PRESSURE

P

ev

*FIG. 4C'*

PRIOR ART

PRIOR ART

*FIG. 4D*

DILATATION

P

ev

*FIG. 4D'*

FIG. 5B

FIG. 5B'

FIG. 5C

FIG. 5C'

FIG. 5D

FIG. 5D'